# FASCICULE DE BREVET EUROPEEN

(11) **EP 4 083 829 B1**
(45) Date de publication et mention de la délivrance du brevet: **19.06.2024**
(21) Numéro de dépôt: 22170166.7
(22) Date de dépôt: 27.04.2022
(51) Int. Cl.: G06F 21/32, A61B 5/1172, G06V 40/13

(54) **DISPOSITIF D' ACQUISITION D' EMPREINTES DIGITALES**
VORRICHTUNG ZUR ERFASSUNG VON FINGERABDRÜCKEN
FINGERPRINT ACQUISITION APPARATUS

(30) Priorité: 30.04.2021 FR 2104541
(43) Date de publication de la demande: 02.11.2022
(73) Titulaire: Idemia Identity & Security France, 92400 Courbevoie (FR)
(72) Inventeur: DUMONT, Denis, 92400 COURBEVOIE (FR); MAILLARD, Sylvain Emile Henri, 92400 COURBEVOIE (FR)
(74) Mandataire: Idemia

(56) Documents cités:
- DE-U1-202004 012 606
- JP-A- 2011 108 128
- US-A1- 2020 342 200

## Description

### DOMAINE DE L'INVENTION

La présente invention concerne un dispositif d'acquisition d'empreintes digitales.

### ETAT DE LA TECHNIQUE

Un dispositif d'acquisition d'empreintes digitales connu comprend une caméra qui acquiert une image d'une empreinte digitale alors qu'un utilisateur a placé un doigt dans un espace d'acquisition en vue de la caméra, situé au-dessus d'une paroi translucide. Pour améliorer la dynamique de l'image, le doigt est éclairé par un éclairage généralement bref et puissant généré par une source d'éclairage interne au dispositif, et traversant la paroi translucide.

Or, cet éclairage est susceptible d'éblouir l'utilisateur veillant à placer son doigt correctement dans l'espace d'acquisition. Par ailleurs, un éclairage ambiant généré par l'environnement extérieur au dispositif est susceptible de traverser la paroi translucide et d'atteindre la caméra, ce qui peut perturber l'acquisition.

Pour surmonter ces problèmes, il a été proposé d'inclure au dispositif une paroi opaque en regard de la première paroi d'acquisition, si bien que l'espace d'acquisition est ménagé entre la paroi translucide et la paroi opaque. En utilisation, la paroi opaque se trouve au-dessus de la première paroi translucide ; autrement dit, l'utilisateur place son doigt ou plus généralement sa main sous la paroi opaque pour que l'empreinte digitale de ce doigt puisse être imagée par la caméra.

La paroi opaque empêche l'utilisateur d'être ébloui par la source d'éclairage interne du dispositif et protège la caméra d'un éclairage ambiant parasitant son fonctionnement.

Or, la paroi opaque empêche l'utilisateur de voir où il passe sa main. L'espace d'acquisition est masqué, ce qui rebute certains utilisateurs qui retiennent leur main. L'impact sur les performances du dispositif est alors très négatif pour une partie de ces utilisateurs.

DE 20 2004 012606 U1 divulgue des systèmes et des procédés permettant d'éviter l'éblouissement de l'utilisateur et d'améliorer la qualité de l'image dans un dispositif de capture biométrique comprenant une caméra et des sources lumineuses. À cette fin, une pluralité de filtres de polarisation sont placés entre l'utilisateur, l'objet à photographier et les sources lumineuses, y compris la lumière ambiante. Les filtres peuvent avoir des directions de polarisation différentes de manière à obtenir l'effet souhaité dans chaque cas.

### EXPOSE DE L'INVENTION

Un but de l'invention est d'acquérir une empreinte digitale au moyen d'un dispositif fiable, tout en étant confortable à utiliser.

Il est à cet effet proposé un dispositif d'acquisition d'empreintes digitales, le dispositif comprenant :
- un espace pour recevoir un doigt d'un utilisateur,
- une source d'éclairage configurée pour émettre une lumière d'éclairage éclairant l'espace,
- une caméra configurée pour acquérir une image d'une empreinte digitale du doigt, lorsque le doigt est reçu dans l'espace,
le dispositif étant caractérisé en ce qu'il comprend un système de polarisation configuré pour :
- polariser dans une première direction la lumière d'éclairage avant que la lumière d'éclairage n'atteigne l'espace,
- polariser dans une deuxième direction la lumière d'éclairage après que la lumière d'éclairage a traversé l'espace, la deuxième direction étant différente de la première direction,
- polariser dans la deuxième direction une lumière ambiante émanant de l'extérieur du dispositif et se propageant vers la caméra, avant que la lumière ambiante n'atteigne l'espace, et
- polariser dans une troisième direction la lumière ambiante après que la lumière ambiante a traversé l'espace, la troisième direction étant différente de la deuxième direction.

Grâce au système de polarisation, la lumière ambiante voit sa puissance fortement diminuer avant d'atteindre la caméra, en raison du fait que celle lumière subit deux polarisations successives dans deux directions de polarisation différentes (la première direction et la deuxième direction). En conséquence, la lumière ambiante n'éblouit pas la caméra, et ne perturbe pas son fonctionnement. En outre, l'utilisateur peut observer l'espace à travers le système de polarisation, ce qui lui permet de l'aider à positionner correctement son doigt, en vue d'une acquisition correcte de son empreinte digitale par la caméra. De surcroît, le système de polarisation a pour avantage de contribuer à une amélioration de la dynamique de l'image montrant l'empreinte digitale acquise par la caméra. Le fonctionnement du dispositif est donc rendu plus fiable.

Le même phénomène de diminution de puissance s'applique à la lumière d'éclairage émise par la source d'éclairage du dispositif, car celle-ci subit également deux polarisations dans deux directions différentes (la deuxième direction et la troisième direction) grâce au système de polarisation. En conséquence, un utilisateur qui regarde l'espace où son doigt est reçu à travers le deuxième polariseur n'est pas ébloui par cette lumière d'éclairage. Ainsi, le dispositif est plus confortable à utiliser.

Le dispositif d'acquisition d'empreintes digitales peut également comprendre les caractéristiques optionnelles suivantes, prises seules ou combinées entre elles à chaque fois que cela fait sens techniquement :
La deuxième direction peut être perpendiculaire à la première direction.

La troisième direction et la première direction peuvent être identiques.

Le système de polarisation peut comprendre un premier polariseur configuré pour :
- polariser la lumière d'éclairage dans la première direction avant que la lumière d'éclairage n'atteigne l'espace, et
- polariser la lumière ambiante dans la première direction après que la lumière ambiante a traversé l'espace.

Le premier polariseur peut délimiter l'espace, de sorte que la lumière d'éclairage atteint l'espace en sortant du premier polariseur.

Le dispositif peut comprendre un boîtier définissant une cavité contenant la source d'éclairage, et une paroi transparente formant une fenêtre entre la cavité et l'espace pour recevoir le doigt, et le premier polariseur peut être fixé à la paroi translucide.

Le dispositif peut comprendre un boîtier définissant une cavité contenant la source d'éclairage, et une paroi transparente formant une fenêtre entre la cavité et l'espace pour recevoir le doigt, le premier polariseur étant agencé dans le boîtier.

Le dispositif peut comprendre un boîtier définissant une cavité contenant la source d'éclairage, le premier polariseur formant une fenêtre transparente séparant la cavité et l'espace pour recevoir le doigt.

Le système de polarisation peut comprendre un deuxième polariseur configuré pour :
- polariser la lumière d'éclairage dans la deuxième direction après que la lumière d'éclairage a traversé l'espace, et
- polariser la lumière ambiante dans la deuxième direction avant que la lumière ambiante n'atteigne l'espace.

Le deuxième polariseur peut délimiter l'espace, de sorte que la lumière ambiante atteint l'espace en sortant du deuxième polariseur.

Le premier polariseur et le deuxième polariseur peuvent s'étendre parallèlement l'un à l'autre.

Le dispositif peut comprendre une deuxième paroi translucide, le deuxième polariseur étant fixé à la deuxième paroi translucide.

Le dispositif peut définir un accès frontal à l'espace et deux accès latéraux à l'espace qui sont opposés l'un à l'autre par rapport à l'accès frontal, de sorte à permettre à l'utilisateur de déplacer le doigt suivant une trajectoire rectiligne, en faisant entrer le doigt dans l'espace par l'un des deux accès latéraux, et en faisant ressortir le doigt de l'espace par l'autre accès latéral.

Le dispositif peut comprendre un boîtier définissant une cavité contenant la source d'éclairage, et une paroi transparente formant une fenêtre entre la cavité et l'espace pour recevoir le doigt, la caméra étant configurée pour acquérir l'image de l'empreinte digitale du doigt, lorsque le doigt est posé sur la paroi transparente.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
La figure 1 et une vue en coupe schématique d'un dispositif d'acquisition d'empreintes digitales selon un premier mode de réalisation de l'invention.
La figure 2 est une vue de dessus du dispositif d'acquisition d'empreintes digitales selon le premier mode de réalisation de l'invention.
La figure 3 illustre une double polarisation que subit une lumière d'éclairage, lorsque celle-ci traverse deux polariseurs.
La figure 4 illustre une double polarisation que subit une lumière ambiante, lorsque celle-ci traverse deux polariseurs.
La figure 5 est une vue en coupe schématique d'un dispositif d'acquisition d'empreintes digitales selon un deuxième mode de réalisation de l'invention.
La figure 6 est une vue en coupe schématique d'un dispositif d'acquisition d'empreintes digitales ne faisant pas partie de l'invention.

Sur l'ensemble des figures, les éléments similaires portent des références identiques.

### DESCRIPTION DETAILLEE DE L'INVENTION

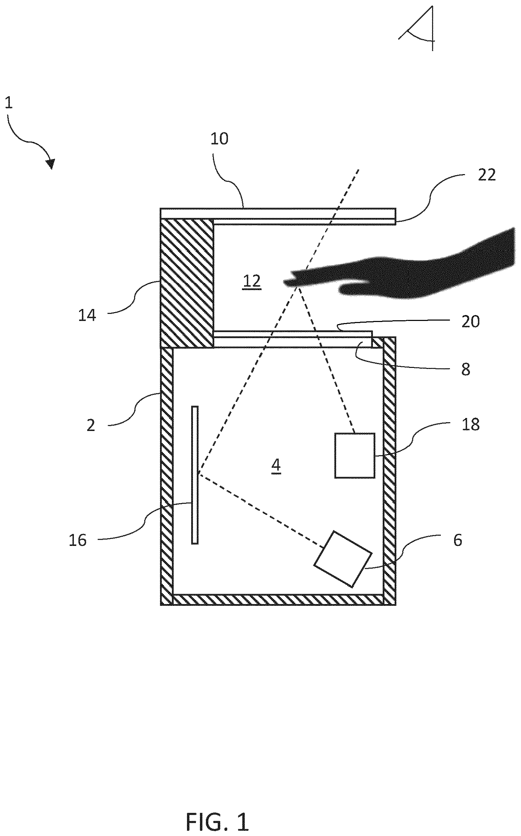

En référence à la **figure 1**, un dispositif d'acquisition d'empreintes digitales 1 comprend un boîtier 2 définissant une cavité interne 4.

Le dispositif d'acquisition d'empreintes digitales 1 comprend en outre une source d'éclairage 6. La source d'éclairage 6 est agencée dans la cavité interne 4.

La source d'éclairage 6 est configurée pour émettre une lumière dans le domaine visible et/ou dans un domaine non-visible (par exemple l'infrarouge).

Par convention, la lumière émise par la source d'éclairage 6 est qualifiée dans le présente texte de « lumière d'éclairage », et, par contraste, une lumière émise par une source extérieure au dispositif d'acquisition d'empreintes est qualifiée de « lumière ambiante ».

Dans le présent texte, un élément qualifié de « translucide » est à interpréter de manière générale comme tout milieu apte à être traversé par de la lumière, autrement dit un matériau non opaque, sans conditions supplémentaires (à moins que de telles conditions soient explicitement indiquées).

Par ailleurs, un élément qualifié de « transparent » dans le présent texte est à interpréter comme un milieu qui est non seulement translucide mais qui laisse également aussi passer la visibilité. Autrement dit, il est possible de voir un objet nettement à travers un élément transparent, alors que ce n'est pas forcément le cas à travers un élément translucide.

Le dispositif d'acquisition d'empreintes digitales 1 comprend une paroi transparente 8.

La paroi transparente 8 forme une partie d'une paroi du boîtier 2, typiquement une paroi supérieure lorsque le dispositif 1 est dans une position d'utilisation. La paroi transparente 8 constitue une fenêtre entre la cavité interne 4 et l'extérieur du boîtier 2. La paroi transparente 8 présente une surface interne dans la cavité interne 4, et une surface externe à l'extérieur du boîtier 2, opposée à la surface interne. De préférence, la lumière d'éclairage ne peut sortir du boîtier 2 que par la paroi transparente 8.

Les surfaces interne et externe de la paroi transparente 8 sont par exemple planes.

La paroi transparente 8 est de préférence transparente et/ou homogène et/ou isotrope. Par exemple, la paroi transparente 8 est en verre.

Le dispositif d'acquisition d'empreintes digitales 1 comprend par ailleurs une paroi translucide 10, agencée en regard et à distance de la paroi transparente 8 de sorte à ménager entre elles un espace 12 où un utilisateur peut placer au moins un de ses doigts.

Par exemple, la hauteur de l'espace 12 ménagé, mesurée comme la distance séparant la paroi transparente 8 et la paroi translucide 10, est comprise entre 3 centimètres et 15 centimètres.

La paroi transparente 8 s'étend entre la cavité interne 4 et l'espace 12.

La paroi translucide 10 présente une surface interne orientée vers l'espace 12, et une surface externe opposée la surface interne. La surface externe débouche à l'extérieur du dispositif d'acquisition d'empreintes digitales 1, dans le sens où elle est visible par un utilisateur, en particulier par un utilisateur en train de placer au moins un de ses doigts dans l'espace 12.

Les surfaces interne et externe de la paroi translucide 10 sont par exemple planes.

La paroi translucide 10 est de préférence transparente et/ou homogène et/ou isotrope, mais ce n'est pas une obligation. Par exemple, la paroi translucide 10 est en polyméthacrylate de méthyle (plexiglas).

Le dispositif d'acquisition d'empreintes digitales 1 comprend par ailleurs une partie de liaison 14 qui relie la paroi translucide 10 à la paroi transparente 8, et faisant office d'espaceur pour maintenir les deux parois 10, 12 à distance l'une de l'autre. Cette partie de liaison 14 peut faire partie du boîtier 2 où être un élément fixé sur le boîtier 2. La paroi translucide 10 s'étend en porte-à-faux depuis cette partie de liaison 14, en regard de la paroi transparente 8. La paroi translucide 10 forme ainsi une casquette.

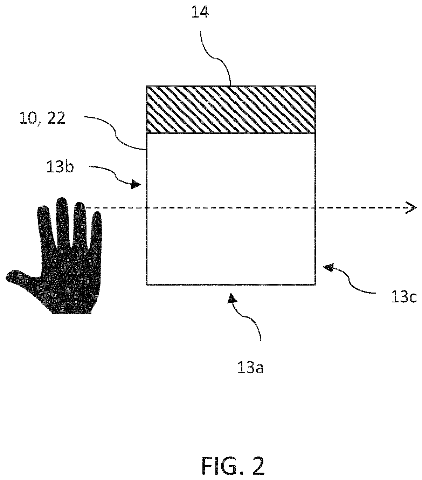

En référence à la figure 2, la partie de liaison 14 est agencée pour ménager un accès frontal 13a à l'espace 12 (qui se trouve sur la figure 1 à droite de l'espace 12 tel que) ainsi que deux accès latéraux 13b, 13c à cet espace 12, qui sont opposés l'un à l'autre par rapport à l'accès frontal 13a. Autrement dit, l'espace 12 est situé entre les deux accès latéraux 13b, 13c, et est par ailleurs situé entre l'accès frontal 13a et la partie de liaison 14. Un utilisateur peut donc fait entrer un doigt dans l'espace 12 via l'accès frontal 13a ou via l'un des accès latéraux 13b, 13c.

Le dispositif d'acquisition d'empreintes digitales 1 est configuré pour que la lumière d'éclairage émise par la source d'éclairage 6 puisse traverser la paroi transparente 8 (sortant ainsi du boîtier 2), puis traverser l'espace 12 ménagé entre les deux parois translucides 8, 10, puis traverser la paroi translucide 10. Le dispositif 1 peut en particulier comprendre un système optique 16 configuré pour guider la lumière émise par la source d'éclairage 6 vers la paroi transparente 8. Dans le mode de réalisation représenté en figure 1, le système optique 16 est formé par un miroir, mais le système optique peut être plus complexe, et peut en variante être absent si la source d'éclairage 6 fait face à la paroi transparente 8.

Le dispositif d'acquisition d'empreintes digitales 1 comprend par ailleurs une caméra 18 configurée pour acquérir des images montrant l'empreinte digitale d'un doigt placé dans l'espace 12. Dans le présent texte, le terme « caméra » n'est pas limité à un moyen d'acquisition d'images sous forme de vidéo ; il est à interpréter dans un sens large, comme couvrant tout moyen d'acquisition d'image, y compris sous forme de vidéo ou d'image unique.

La caméra 18 est agencée dans la cavité interne 4 du boîtier 2, de sorte à pouvoir recevoir de la lumière émanant de l'espace 12. La caméra 12 est en particulier configurée pour acquérir une image d'un doigt reçu dans l'espace 12.

La caméra 18 est en particulier configurée pour réaliser une telle acquisition alors que le doigt est en suspension dans l'espace 12, sans que celui-ci ne touche le dispositif 1. Ceci est avantageux par rapport aux dispositifs d'acquisition d'empreintes digitales requérant la mise en contact d'un doigt sur une surface d'acquisition, car un tel contact est susceptible de déformer l'empreinte digitale, ce qui peut conduire à une acquisition imparfaite de cette empreinte.

Le dispositif 1 peut comprendre un système optique (non illustré) pour rediriger la lumière émanant de l'espace 12 vers la caméra 18.

La caméra 18 est apte à communiquer avec une unité de traitement d'image configurée pour mettre en oeuvre toute ou partie d'un procédé d'authentification connu de l'état de la technique. Par exemple, ce procédé d'authentification peut avoir pour but d'identifier l'utilisateur ayant placé son doigt dans l'espace 12, ou de déterminer si cet utilisateur a le droit ou non d'accéder à un service ou une zone.

Il est à noter que cette unité de traitement d'image peut être comprise dans le dispositif d'acquisition d'empreintes digitales 1, mais cela n'est aucunement requis car cette unité peut être située à l'extérieur de ce dispositif 1. Dans le second cas, le dispositif d'acquisition d'empreintes digitales 1 peut comprendre une interface de communication pour communiquer avec un dispositif externe, tel qu'un serveur, pourvu d'une telle unité de traitement d'image.

Le dispositif d'acquisition d'empreintes digitales 1 comprend par ailleurs un système de polarisation. De manière générale, le système de polarisation est configuré pour
- polariser dans une première direction la lumière d'éclairage avant que la lumière d'éclairage n'atteigne l'espace 12,
- polariser dans une deuxième direction la lumière d'éclairage après que la lumière d'éclairage a traversé l'espace 12, la deuxième direction étant différente de la première direction,
- polariser dans la deuxième direction une lumière ambiante émanant de l'extérieur du dispositif 1 et se propageant vers la caméra 18, avant que la lumière ambiante n'atteigne l'espace 12, et
- polariser dans une troisième direction la lumière ambiante après que la lumière ambiante a traversé l'espace 12, la troisième direction étant différente de la deuxième direction.

Dans le premier mode de réalisation représenté en figure 1, le système de polarisation comprend un premier polariseur 20 configuré pour polariser une lumière dans une première direction.

Le premier polariseur 20 est transparent. Le premier polariseur est agencé pour être traversé par la lumière d'éclairage émise par la source d'éclairage 6, et ce avant que la lumière d'éclairage n'atteigne l'espace 12.

Le premier polariseur 20 est par ailleurs agencé pour être traversé par la lumière ambiante émise par une source extérieure au dispositif 1, et ce avant que cette lumière ambiante n'atteigne la caméra 18. Ainsi, dans ce mode de réalisation, la première direction et la troisième direction sont identique.

Dans le premier mode de réalisation illustré sur la figure 1, le premier polariseur 20 délimite l'espace 12, de sorte que la lumière d'éclairage en provenance de la source d'éclairage 6 atteint l'espace 12 en sortant du premier polariseur 20. Ceci permet de garantir que le premier polariseur 20 est le dernier élément optique du dispositif qui modifie les propriétés de la lumière émanant de la source d'éclairage 6 avant que cette lumière n'atteigne l'espace 12. Si des éléments optiques du dispositif tels que la paroi transparente 8 étaient situés en aval du premier polariseur 20, ils pourraient altérer la polarisation de la lumière d'éclairage, si bien que la lumière d'éclairage ne serait plus parfaitement dans la première direction de polarisation lorsqu'elle atteint l'espace 12. Ce problème est évité dans ce premier mode de réalisation.

En pratique, le premier polariseur 20 est placé à l'extérieur du boîtier 2, entre la paroi transparente 8 est l'espace 12.

Le premier polariseur 20 est fixé à la paroi transparente 8. Plus précisément, le premier polariseur 20 est fixé à la surface externe de la paroi transparente 8.

Le premier polariseur 20 se présente par exemple sous la forme d'un film fixé sur la paroi transparente 8 à l'aide d'un adhésif.

Dans le premier mode de réalisation illustré sur la figure 1, le système de polarisation comprend en outre un deuxième polariseur 22. Le premier polariseur 20 est configuré pour polariser la lumière dans une deuxième direction différente de la première polarisation, après que la lumière a traversé l'espace 12. L'espace 12 est situé entre le premier polariseur 20 et le deuxième polariseur 22.

Le deuxième polariseur est translucide, voire transparent.

Le deuxième polariseur 22 délimite l'espace 12, de sorte que la lumière ambiante atteint l'espace 12 en sortant du deuxième polariseur 22. Ceci permet de garantir que le deuxième polariseur 22 est le dernier élément optique du dispositif qui modifie les propriétés de la lumière ambiante émanant de l'extérieur du dispositif 1, avant son entrée dans l'espace 12 d'éclairage.

En pratique, le deuxième polariseur 22 est fixé à la paroi translucide 10. Plus précisément, le deuxième polariseur 22 est fixé à la surface interne de la paroi translucide 10. De la sorte, la paroi translucide 10 protège le deuxième polariseur 22 d'agressions extérieures au dispositif 1, qui risquerait de l'abîmer et ainsi altérer sa fonction de polarisation.

Le deuxième polariseur 22 se présente par exemple sous la forme d'un film fixé sur la paroi translucide 10 à l'aide d'un adhésif.

Le premier polariseur 20 et le deuxième polariseur 22 s'étendent parallèlement l'un à l'autre.

Comme indiqué précédemment, la deuxième direction de polarisation est différente de la première direction de polarisation. De manière préférentielle, l'angle entre la première direction de polarisation et la deuxième direction de polarisation est compris entre 70 et 110 degrés, voire est compris entre 80 et 100 degrés. De manière encore plus préférentielle, la deuxième direction de polarisation est perpendiculaire à la première direction de polarisation.

Un procédé d'acquisition d'empreintes digitales au moyen du dispositif d'acquisition d'empreintes digitales 1 comprend les étapes suivantes.

On suppose que le dispositif d'acquisition d'empreintes digitales 1 est placé dans une position d'utilisation, dans laquelle la paroi transparente 8 forme une portion de paroi supérieure du boîtier 2 et dans laquelle la paroi translucide 10 se trouve au-dessus de la paroi transparente 8.

Un utilisateur se place devant le dispositif 1, et en particulier en face de l'accès frontal 13a à l'espace 12. Le dispositif 1 est typiquement placé à une hauteur permettant à l'utilisateur de surplomber le dispositif 1, de sorte que le deuxième polariseur 20 se trouve entre l'espace 12 et les yeux de l'utilisateur.

L'utilisateur fait pénétrer au moins une de ses doigts dans l'espace 12 ménagé entre les polariseurs 20 et 22, de sorte que l'empreinte digitale d'un doigt soit en vue de la caméra 18.

L'utilisateur peut en particulier faire pénétrer un doigt par un des accès latéraux à l'espace 12, par exemple l'accès latéral 13b, et le déplacer vers l'autre accès latéral 13c selon une trajectoire rectiligne comme représenté en figure 2.

La source d'éclairage 6 émet la lumière d'éclairage discutée précédemment, afin d'éclairer l'espace 12 et en particulier le doigt que l'utilisateur y a placé. L'émission de la lumière d'éclairage est par exemple déclenchée sur une détection de présence du doigt dans le champ de vision de la caméra 18, à l'aide de moyens de détection adéquats du dispositif d'acquisition d'empreintes digitales 1.

La lumière d'éclairage est émise typiquement sous la forme d'une succession de flashs brefs et puissants.

La lumière d'éclairage se propage dans la cavité interne 4, est redirigée vers la paroi transparente 8 par le système optique 16, et traverse la paroi transparente 8. La lumière d'éclairage traverse par ailleurs le premier polariseur 20, lequel polarise la lumière d'éclairage dans la première direction. Le premier polariseur 20 agit sur la lumière d'éclairage comme un filtre : toute direction de polarisation éventuellement présente dans la lumière d'éclairage en amont du premier polariseur 20 est éliminée lors de la traversée du premier polariseur 20 par cette lumière, à l'exception de la première direction.

Une fois polarisée dans la première direction par le premier polariseur 20, la lumière d'éclairage se propage dans l'espace 12.

Une partie de la lumière d'éclairage polarisée dans la première direction éclaire en particulier l'empreinte digitale du doigt placé dans l'espace 12. Cette partie de la lumière d'éclairage est réfléchie par le doigt de l'utilisateur, et pénètre à nouveau dans le boîtier 2 en traversant le premier polariseur 20 et la paroi transparente 8, avant d'atteindre la caméra 18.

La caméra 18 acquiert une image montrant l'empreinte digitale du doigt reçu dans l'espace 12. Cette image peut ensuite être utilisée dans le cadre du procédé d'authentification cité précédemment.

Pendant cette acquisition, il n'est pas nécessaire que le doigt soit mis en contact avec le dispositif 1, en particulier avec la paroi transparente 8 ou le premier polariseur 20.

Comme l'utilisateur peut voir l'espace 12 à travers la paroi translucide 10 et à travers le deuxième polariseur 22, l'utilisateur a pu positionner très facilement son doigt dans l'espace 12 en regard de la caméra 18, ce a permis de raccourcir la durée de mise en oeuvre du procédé. Par contraste, un utilisateur incapable de visualiser où se trouve son doigt par rapport à la paroi transparente 8 aurait tendance à hésiter ou en tout cas tâtonner pour placer correctement son doigt, ce qui ne pourrait que retarder le déclenchement de l'étape d'acquisition ou conduire à l'obtention d'une image ne montrant pas l'empreinte digitale correctement, compromettant ainsi le bon fonctionnement du procédé qui exploite ensuite cette image.

Bien entendu, l'utilisation de la source d'éclairage 6 améliore la lisibilité de l'empreinte digitale dans l'image acquise par la caméra 18. Le premier polariseur 20 améliore en outre la dynamique de cette image.

On a vu précédemment qu'une portion de la lumière d'éclairage polarisée dans la première direction a été réfléchie par le doigt. Une partie restante de la lumière d'éclairage polarisée dans la première direction, non bloquée par le doigt ou tout autre éventuel obstacle, traverse entièrement l'espace 12 en direction du deuxième polariseur 22 et de la paroi translucide 10, puis traverse le deuxième polariseur 22. Cette partie restante subit donc une nouvelle polarisation, mais cette fois dans la deuxième direction, différente de la première direction. Ainsi, le deuxième polariseur applique à la partie restante de la lumière d'éclairage un second filtrage éliminant une grande partie de la puissance transportée par cette lumière. En conséquence, l'utilisateur regardant en direction de l'espace 12 à travers le deuxième polariseur 22 n'est pas ébloui par la lumière d'éclairage.

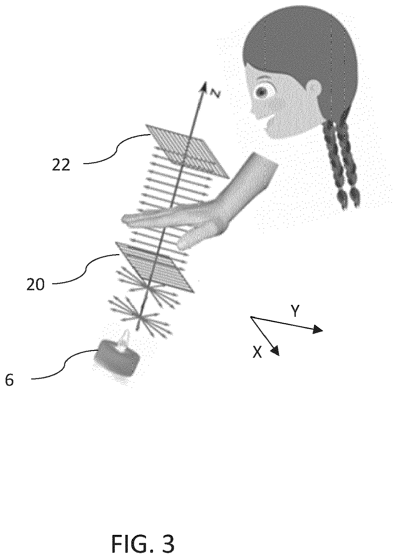

On a représenté en figure 3 de manière schématique un exemple de double polarisation de la lumière d'éclairage réalisée par les deux polariseurs 20 et 22. Dans cet exemple, le premier polariseur 20 polarise la lumière d'éclairage dans une direction parallèle à un axe X, et le deuxième polariseur polarise la lumière d'éclairage dans une direction parallèle à un axe Y perpendiculaire à l'axe X. Dans cette situation de perpendicularité, la lumière d'éclairage est complètement éliminée par les deux polariseurs 20, 22 avant d'atteindre l'utilisateur.

Considérons à présent une lumière ambiante émise par une source externe au dispositif d'acquisition d'empreintes digitales 1. Une partie de cette lumière ambiante peut traverser la paroi translucide 10 et le deuxième polariseur 22, puis traverser l'espace 12, traverser la paroi transparente 8 et le première polariseur (en supposant qu'elle n'est pas bloquée par un doigt ou tout autre obstacle placé dans l'espace 12). Cette partie de la lumière ambiante subit donc deux polarisations successives avant d'atteindre la caméra 18 : une polarisation dans la deuxième direction, puis une polarisation dans la première direction. De la sorte, une grande partie de la puissance de la lumière ambiante est éliminée avant d'atteindre la caméra 18, ce qui a pour conséquence de limiter voire éliminer les perturbations que causerait la lumière ambiante sur l'acquisition d'image réalisée par la caméra 18.

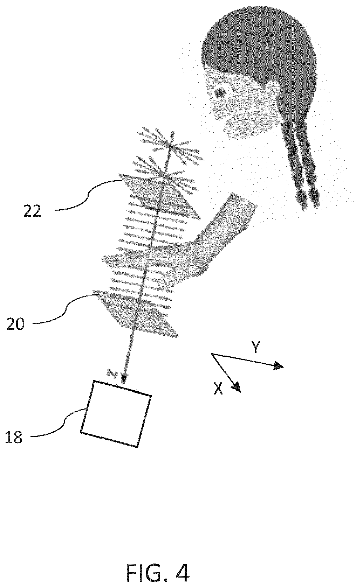

On a représenté en figure 4 de manière schématique la double polarisation de la lumière ambiante par les deux polariseurs 20 et 22 dans le même exemple de réalisation que celui de la figure 3. Dans cet exemple, la lumière ambiante est complètement éliminée par les deux polariseurs 20, 22 avant d'atteindre la caméra 6.

La lumière ambiante permet en outre à l'utilisateur regardant en direction de la surface supérieure de la paroi translucide 10 de pouvoir contempler l'espace 12 où il a placé son doigt.

En définitive, les deux polariseurs 20, 22 permettent d'atteindre un double objectif : réduire les risques d'éblouir un utilisateur veillant à bien positionner son doigt en vue de l'acquisition de son empreinte digitale par le dispositif 1, et les risques d'éblouir la caméra 18. Dans le cas particulier où la première direction de polarisation et la deuxième direction de polarisation sont perpendiculaires, l'utilisateur et la caméra 18 voient tous deux le doigt reçu dans l'espace, mais ne se voient pas mutuellement, ce qui est très avantageux car les risques d'éblouissement précités sont totalement éliminés.

Il a été pris dans ce qui précède l'exemple de l'acquisition de l'image d'un doigt d'un utilisateur. Toutefois, le dispositif 1 peut tout à fait être utilisé pour acquérir une ou plusieurs images d'empreintes digitales de plusieurs doigts de l'utilisateur. Pour cela, l'utilisateur peut faire entrer plusieurs de ses doigts dans l'espace 12, de sorte que les doigts traversent ensemble l'espace 12 en allant d'un accès latéral à l'autre, en suivant une trajectoire rectiligne comme cela est représenté par une flèche en pointillés sur la figure 2. Au cours de cette traversée, les doigts sont successivement ou simultanément en vue de la caméra 18, si bien qu'une ou plusieurs images les montrant peuvent être acquises par la caméra 18.

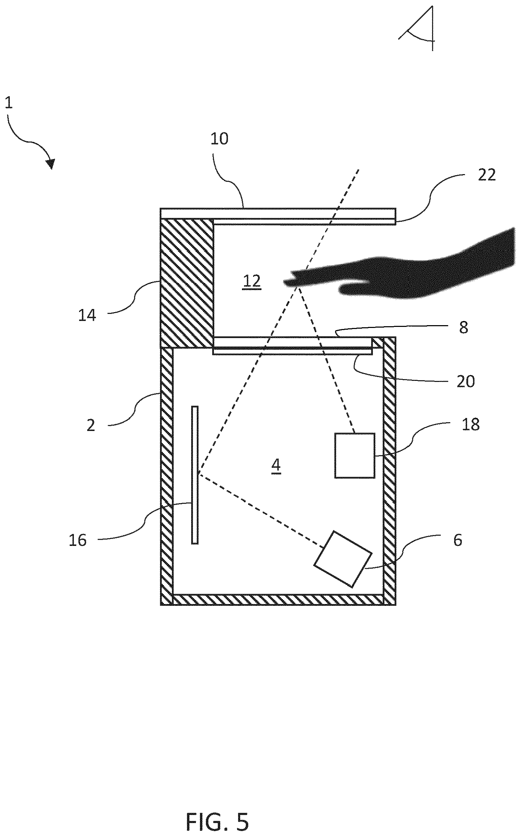

Est représenté en figure 5 un deuxième mode de réalisation du dispositif 1, qui diffère du premier mode de réalisation représenté en figure 1 par la forme de son système de polarisation : en effet, le premier polariseur 20 est situé dans la cavité interne 4 du boîtier 2. Ceci permet de protéger le premier polariseur 20 d'agressions en provenance de l'extérieur du boîtier 2. Par exemple, le premier polariseur 20 est fixé à la surface interne de la paroi transparente 8. En contrepartie de cet effet protecteur, le filtrage de lumière réalisée par les deux polarisateurs 20 et 22 peut être légèrement moins performant que dans le premier mode de réalisation, car la première paroi translucide peut « dépolariser » la lumière d'éclairage ayant été polarisée dans la première direction, avant son entrée dans l'espace 12 et sa polarisation ultérieure par le deuxième polariseur 22.

Dans les deux modes de réalisation décrits précédemment, le système de polarisation ne comprend que deux polariseurs. En particulier, le premier polariseur 20 se charge non seulement de polariser la lumière émanant de la source d'éclairage 6, mais également la lumière allant vers la caméra 6. Cette configuration présente l'avantage d'être simple de fabrication.

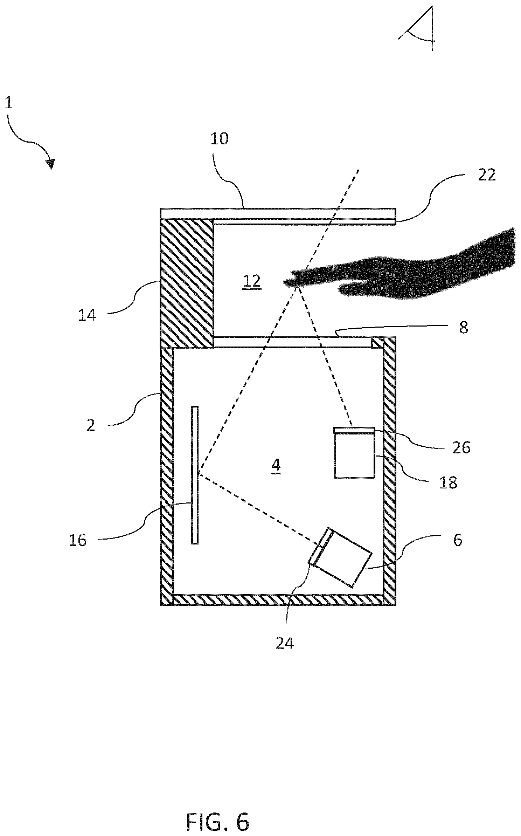

Est représenté en figure 6 un dispositif 1 qui ne fait pas partie de l'invention, et qui diffère du deuxième mode de réalisation par le fait que le système de polarisation comprend trois polariseurs. Plus précisément, le premier polariseur 20 du deuxième mode de réalisation est remplacé par une paire de polariseurs 24, 26 distincts.

D'une part, le polariseur 24 est configuré pour polariser dans la première direction la lumière émanant de la source d'éclairage 6 avant que celle-ci n'atteigne l'espace 12. Le polariseur 24 est agencé dans la cavité interne 4. Le polariseur 24 est fixé par exemple à la source d'éclairage 6, mais peut en variante être à distance de celle-ci. Le polariseur 24 est translucide, voire transparent.

D'autre part, le polariseur 26 est configuré pour polariser la lumière en provenance de l'espace 4 dans une troisième direction, avant que celle-ci n'atteigne la caméra. Le polariseur 26 est également agencé dans la cavité interne 4. Le polariseur 26 est fixé par exemple à la caméra 18, mais peut en variante être à distance de celle-ci. Le polariseur 26 est transparent.

La troisième direction est de préférence identique à la première direction, afin d'optimiser les performances d'atténuation du système de polarisation, mais pas obligatoirement.

Un avantage du dispositif de la figure 6 est que la paire de polariseurs 24, 26 peuvent être de dimensions réduites. Ainsi le coût de la paire de polariseurs 24, 26 peut être inférieur au coût du polariseur 20.

D'autres modes de réalisation du dispositif d'acquisition d'empreintes digitales 1 non illustrés sont également envisageables. Ces modes de réalisation peuvent comprendre les caractéristiques exposées ci-dessous.

Le premier polariseur 20 peut être positionné dans le boîtier 2, à distance de la paroi transparente 8.

Le deuxième polariseur 22 peut par ailleurs être fixé sur la surface externe de la paroi translucide 10, ou à distance de la paroi translucide 10.

Il n'est pas du tout obligatoire que les deux polariseurs soient alignés, pourvu qu'ils puissent être successivement traversés par la lumière d'éclairage émise par la source d'éclairage 6, et être successivement traversés par de la lumière ambiante émanant de l'extérieur, avant que cette lumière ne puisse atteindre la caméra 18.

Dans les modes de réalisation représenté sur les figures 1 et 5, les polariseurs 20, 22 et les deux parois translucides 8, 10 constituent quatre éléments translucides qui sont successivement traversés par la lumière d'éclairage, et qui peuvent être successivement traversés par une lumière ambiante émanant de l'extérieur du dispositif 1. Toutefois, il est entendu que le dispositif 1 peut parfaitement ne comprendre que deux éléments translucides assurant les fonctions de polarisation précitées et par ailleurs délimitant entre elles l'espace 12 où les doigts d'utilisateurs sont reçus.

L'invention est définie par les revendications ci-après.

## Revendications

1. Dispositif (1) d'acquisition d'empreintes digitales sans contact, le dispositif (1) comprenant :
- un boîtier (2) définissant une cavité (4),
- un espace (12) pour recevoir un doigt d'un utilisateur,
- une paroi transparente (8) formant une fenêtre entre la cavité (4) et l'espace (12) pour recevoir le doigt,
- une paroi translucide (10) agencée en regard et à distance de la paroi transparente (8) de sorte à ménager entre elles l'espace (12),
- une source d'éclairage (6) contenue dans la cavité (4) et configurée pour émettre une lumière d'éclairage éclairant l'espace (12),
- une caméra (18) configurée pour acquérir une image d'une empreinte digitale du doigt, lorsque le doigt est reçu dans l'espace (12) sans entrer en contact avec le dispositif (1),
le dispositif comprenant en outre un système de polarisation (20, 22) configuré pour :
- polariser dans une première direction la lumière d'éclairage avant que la lumière d'éclairage n'atteigne l'espace (12),
- polariser dans une deuxième direction la lumière d'éclairage après que la lumière d'éclairage a traversé l'espace (12), la deuxième direction étant différente de la première direction,
- polariser dans la deuxième direction une lumière ambiante émanant de l'extérieur du dispositif (1) et se propageant vers la caméra (18), avant que la lumière ambiante n'atteigne l'espace (12), et
- polariser dans la première direction la lumière ambiante après que la lumière ambiante a traversé l'espace (12),
dans lequel le système de polarisation (20, 22) comprend un premier polariseur (20) formant ou étant fixé à la paroi transparente (8), et configuré pour :
- polariser la lumière d'éclairage dans la première direction avant que la lumière d'éclairage n'atteigne l'espace (12), et
- polariser la lumière ambiante dans la première direction après que la lumière ambiante a traversé l'espace (12), et avant que la lumière ambiante n'atteigne la caméra,
dans lequel le système de polarisation comprend en outre un deuxième polariseur (22) fixé à la paroi translucide (10), et configuré pour :
- polariser la lumière d'éclairage dans la deuxième direction après que la lumière d'éclairage a traversé l'espace (12), et
- polariser la lumière ambiante dans la deuxième direction avant que la lumière ambiante n'atteigne l'espace (12).

2. Dispositif (1) selon la revendication précédente, dans lequel la deuxième direction est perpendiculaire à la première direction.

3. Dispositif (1) selon la revendication précédente, dans lequel le premier polariseur (20) délimite l'espace (12), de sorte que la lumière d'éclairage atteint l'espace (12) en sortant du premier polariseur (20).

4. Dispositif (1) selon l'une des revendications précédentes, comprenant un boîtier (2) définissant une cavité (4) contenant la source d'éclairage (6), et une paroi transparente (8) formant une fenêtre entre la cavité (4) et l'espace (12) pour recevoir le doigt, dans lequel le premier polariseur (20) est agencé dans le boîtier (2).

5. Dispositif (1) selon l'une quelconque des revendications précédentes, dans lequel le deuxième polariseur (22) délimite l'espace (12), de sorte que la lumière ambiante atteint l'espace (12) en sortant du deuxième polariseur (22).

6. Dispositif (1) selon l'une quelconque des revendications précédentes, dans lequel le premier polariseur (20) et le deuxième polariseur (22) s'étendent parallèlement l'un à l'autre.

7. Dispositif (1) selon l'une des revendications précédentes, définissant un accès frontal (13a) à l'espace (12) et deux accès latéraux (13b, 13c) à l'espace (12) qui sont opposés l'un à l'autre par rapport à l'accès frontal (13a), de sorte à permettre à l'utilisateur de déplacer le doigt suivant une trajectoire rectiligne, en faisant entrer le doigt dans l'espace (12) par l'un des deux accès latéraux (13b), et en faisant ressortir le doigt de l'espace par l'autre accès latéral (13c).

8. Dispositif (1) selon l'une des revendications précédentes, comprenant un boîtier (2) définissant une cavité (4) contenant la source d'éclairage (6), et une paroi transparente (8) formant une fenêtre entre la cavité (4) et l'espace (12) pour recevoir le doigt, dans lequel la caméra est configurée pour acquérir l'image de l'empreinte digitale du doigt, lorsque le doigt est posé sur la paroi transparente (8).

## Patentansprüche

1. Vorrichtung (1) zur kontaktlosen Erfassung von Fingerabdrücken, wobei die Vorrichtung (1) Folgendes umfasst:
- ein Gehäuse (2), das einen Hohlraum (4) definiert,
- einen Raum (12) zur Aufnahme eines Fingers eines Benutzers,
- eine transparente Wand (8), die ein Fenster zwischen dem Hohlraum (4) und dem Raum (12) zur Aufnahme des Fingers bildet,
- eine durchscheinende Wand (10), die gegenüber und in einem Abstand von der transparenten Wand (8) angeordnet ist, um zwischen ihnen den Raum (12) zu schaffen,
- eine Beleuchtungsquelle (6), die in dem Hohlraum (4) enthalten und dazu ausgebildet ist, ein Beleuchtungslicht auszusenden, das den Raum (12) beleuchtet,
- eine Kamera (18), die dazu ausgebildet ist, ein Bild eines Fingerabdrucks des Fingers zu erfassen, wenn der Finger in dem Raum (12) aufgenommen ist, ohne mit der Vorrichtung (1) in Kontakt zu kommen,
wobei die Vorrichtung ferner ein Polarisierungssystem (20, 22) umfasst, das dazu ausgebildet ist:
- das Beleuchtungslicht in einer ersten Richtung zu polarisieren, bevor das Beleuchtungslicht den Raum (12) erreicht,
- das Beleuchtungslicht in einer zweiten Richtung zu polarisieren, nachdem das Beleuchtungslicht den Raum (12) durchquert hat, wobei sich die zweite Richtung von der ersten Richtung unterscheidet,
- in der zweiten Richtung ein Umgebungslicht zu polarisieren, das von außerhalb der Vorrichtung (1) kommt und sich zur Kamera (18) hin ausbreitet, bevor das Umgebungslicht den Raum (12) erreicht, und
- das Umgebungslicht in der ersten Richtung zu polarisieren, nachdem das Umgebungslicht den Raum (12) durchquert hat,
wobei das Polarisierungssystem (20, 22) einen ersten Polarisator (20) umfasst, der die transparente Wand (8) bildet oder an ihr befestigt ist und dazu ausgebildet ist:
- das Beleuchtungslicht in der ersten Richtung zu polarisieren, bevor das Beleuchtungslicht den Raum (12) erreicht, und
- das Umgebungslicht in der ersten Richtung zu polarisieren, nachdem das Umgebungslicht den Raum (12) durchquert hat und bevor das Umgebungslicht die Kamera erreicht,
wobei das Polarisierungssystem ferner einen zweiten Polarisator (22) umfasst, der an der durchscheinenden Wand (10) befestigt ist und dazu ausgebildet ist:
- das Beleuchtungslicht in der zweiten Richtung zu polarisieren, nachdem das Beleuchtungslicht den Raum (12) durchquert hat, und
- das Umgebungslicht in der zweiten Richtung zu polarisieren, bevor das Umgebungslicht den Raum (12) erreicht.

2. Vorrichtung (1) nach dem vorhergehenden Anspruch, wobei die zweite Richtung senkrecht zur ersten Richtung verläuft.

3. Vorrichtung (1) nach dem vorhergehenden Anspruch, wobei der erste Polarisator (20) den Raum (12) so begrenzt, dass das Beleuchtungslicht den Raum (12) beim Austritt aus dem ersten Polarisator (20) erreicht.

4. Vorrichtung (1) nach einem der vorhergehenden Ansprüche, umfassend ein Gehäuse (2), das einen Hohlraum (4) definiert, der die Beleuchtungsquelle (6) enthält, und eine transparente Wand (8), die ein Fenster zwischen dem Hohlraum (4) und dem Raum (12) zur Aufnahme des Fingers bildet, wobei der erste Polarisator (20) im Gehäuse (2) angeordnet ist.

5. Vorrichtung (1) nach einem der vorhergehenden Ansprüche, wobei der zweite Polarisator (22) den Raum (12) so begrenzt, dass das Umgebungslicht den Raum (12) beim Austritt aus dem zweiten Polarisator (22) erreicht.

6. Vorrichtung (1) nach einem der vorhergehenden Ansprüche, wobei sich der erste Polarisator (20) und der zweite Polarisator (22) parallel zueinander erstrecken.

7. Vorrichtung (1) nach einem der vorhergehenden Ansprüche, die einen vorderen Zugang (13a) zu dem Raum (12) und zwei Seitenzugänge (13b, 13c) zu dem Raum (12), die bezogen auf den vorderen Zugang (13a) einander gegenüberliegen, definiert, um es dem Benutzer zu ermöglichen, den Finger eine geradlinige Bahn entlang zu bewegen, indem der Finger durch einen der zwei Seitenzugänge (13b) in den Raum (12) eingeführt und durch den anderen Seitenzugang (13c) wieder aus dem Raum herausgeführt wird.

8. Vorrichtung (1) nach einem der vorhergehenden Ansprüche, umfassend ein Gehäuse (2), das einen Hohlraum (4) definiert, der die Beleuchtungsquelle (6) enthält, und eine transparente Wand (8), die ein Fenster zwischen dem Hohlraum (4) und dem Raum (12) zur Aufnahme des Fingers bildet, wobei die Kamera dazu ausgebildet ist, das Bild des Fingerabdrucks des Fingers zu erfassen, wenn der Finger auf die transparente Wand (8) gelegt wird.

## Claims

1. Contactless fingerprint acquisition apparatus (1), the apparatus (1) comprising:
- a casing (2) defining a cavity (4);
- a space (12) for receiving a finger of a user;
- a transparent wall (8) forming a window between the cavity (4) and the space (12) for receiving the finger;
- a translucent wall (10) arranged opposite and remote from the transparent wall (8) so as to provide the space (12) between them;
- a lighting source (6) contained in the cavity (4) and configured to emit illumination light illuminating the space (12);
- a camera (18) configured to acquire an image of a fingerprint of the finger, when the finger is received in the space (12), without coming into contact with the apparatus (1);
the apparatus further comprising a polarization system (20, 22) configured to:
- polarize the illumination light in a first direction before the illumination light reaches the space (12);
- polarize the illumination light in a second direction after the illumination light has passed through the space (12), with the second direction being different from the first direction;
- polarize ambient light in the second direction emanating from outside the apparatus (1) and propagating towards the camera (18), before the ambient light reaches the space (12); and
- polarize the ambient light in the first direction after the ambient light has passed through the space (12), wherein the polarization system (20, 22) comprises a first polarizer (20) forming or being attached to the transparent wall (8) and being configured to:
- polarize the illumination light in the first direction before the illumination light reaches the space (12); and
- polarize the ambient light in the first direction after the ambient light has passed through the space (12), and before the ambient light reaches the camera;
wherein the polarization system further comprises a second polarizer (22) attached to the translucent wall (10) and being configured to:
- polarize the illumination light in the second direction after the illumination light has passed through the space (12); and
- polarize the ambient light in the second direction before the ambient light reaches the space (12).

2. Apparatus (1) according to the preceding claim, wherein the second direction is perpendicular to the first direction.

3. Apparatus (1) according to the preceding claim, wherein the first polarizer (20) delimits the space (12), so that the illumination light reaches the space (12) upon exiting the first polarizer (20).

4. Apparatus (1) according to any of the preceding claims, comprising a casing (2) defining a cavity (4) containing the lighting source (6), and a transparent wall (8) forming a window between the cavity (4) and the space (12) for receiving the finger, wherein the first polarizer (20) is arranged in the casing (2).

5. Apparatus (1) according to any one of the preceding claims, wherein the second polarizer (22) delimits the space (12), so that the ambient light reaches the space (12) upon exiting the second polarizer (22).

6. Apparatus (1) according to any one of the preceding claims, wherein the first polarizer (20) and the second polarizer (22) extend parallel to each other.

7. Apparatus (1) according to any of the preceding claims, defining a front access (13a) to the space (12) and two lateral accesses (13b, 13c) to the space (12) that are opposite each other relative to the front access (13a), so as to allow the user to move the finger along a rectilinear trajectory, by inserting the finger into the space (12) via one of the two lateral accesses (13b), and by removing the finger from the space via the other lateral access (13c).

8. Apparatus (1) according to any of the preceding claims, comprising a casing (2) defining a cavity (4) containing the lighting source (6), and a transparent wall (8) forming a window between the cavity (4) and the space (12) for receiving the finger, wherein the camera is configured to acquire the image of the fingerprint of the finger, when the finger is placed on the transparent wall (8).
